# EUROPEAN PATENT APPLICATION

(11) **EP 3 944 896 A1**
(43) Date of publication of application: **02.02.2022**
(21) Application number: 21188344.2
(22) Date of filing: 28.07.2021
(51) Int. Cl.: B01L 3/00, C12Q 1/68, B01J 19/00

(54) **MULTIPLEX PCR CHIP AND MULTIPLEX PCR METHOD USING SAME**

(30) Priority: 30.07.2020 KR 20200095509
(71) Applicant: Genesystem Co., Ltd., Yuseong-gu Daejeon 34028 (KR)
(72) Inventor: SEO, Yu Jin, 14295 Gyeonggi-do (KR); CHOI, Ok Ran, 35211 Daejeon (KR); LEE, Dobu, 28165 Cheongju-si (KR); PARK, Ji Young, 34052 Daejeon (KR)
(74) Representative: Scheele Wetzel Patentanwälte

(57) **Abstract**

A multiplex PCR chip capable of simultaneously detecting multiple target genes and a multiplex PCR method using the same are proposed. More specifically, in the multiplex PCR chip and multiplex PCR method, after a plurality of spatially separated particle-forming grooves is formed in one or more reaction chambers and a probe in a solution state is injected into the particle-forming grooves, planar shapes of the particle-forming grooves are varied or shapes and patterns of particle holders respectively formed on inner surfaces of the particle-forming grooves are varied, and the probe including primers specifically hybridizing with sequences of different nucleic acid molecules is injected into the particle-forming grooves, whereby simultaneous multiplex detection is possible by allowing multiple target genes to be detected on the basis of positions and shapes of the probe particles and the shapes and patterns of the particle holders respectively formed inside of the probe particles.

## Description

### CROSS REFERENCE TO RELATED APPLICATION

The present application claims priority to Korean Patent Application No. 10-2020-0095509, filed July 30, 2020, the entire contents of which is incorporated herein for all purposes by this reference.

### BACKGROUND OF THE INVENTION

### Field of the Invention

The present invention relates to a multiplex PCR chip capable of simultaneously detecting a plurality of target genes and a multiplex PCR method using the same.

### Description of the Related Art

Polymerase Chain Reaction (PCR) is a technique in which a sample solution containing nucleic acid molecules is repeatedly heated and cooled and a region (i.e., target nucleic acids) having specific nucleotide sequences is replicated in chains and exponentially amplified, and is the technique that enables target genes to be detected by amplifying and replicating trace amounts of the target nucleic acids.

In such a PCR method, a DNA denaturation step, an annealing step, and a DNA extension step are repeatedly performed, wherein the DNA denaturation step is a step of separating double-stranded DNA into single-stranded DNA by heating the sample solution containing double-stranded template DNA at a specific temperature, for example, about 95°C for 5 seconds, the annealing step is a step of injecting primers having sequences complementary to specific nucleotide sequences to be amplified into the sample solution after the DNA denaturation step, and hybridizing the primers to the specific nucleotide sequences of the single-stranded DNA together with the single-stranded DNA by maintaining a specific temperature, for example, 50°C for 5 seconds so as to form a partial DNA-primer complex, and the DNA extension step is a step of maintaining the sample solution at an activation temperature of the DNA polymerase, for example, 72°C for 5 seconds after the annealing step and forming, by DNA polymerase, the double-stranded DNA on the basis of the primer of the partial DNA-primer complex. In addition, the target nucleic acids may be detected by way of detecting fluorescent light generated from a fluorescent dye combined with the target nucleic acids.

In order to properly perform such a PCR method, there are required devices as well as materials, including: a reagent (i.e., primer and fluorescent dye); a sample (i.e., sample solution); a PCR chip provided with one or more reaction chambers; and a PCR device configured to heat and cool the PCR chip to induce an amplification reaction and measure the results. In particular, a small and flat micro-PCR chip is used in a portable real-time PCR device. The micro-PCR chip is provided with one or more reaction chambers capable of accommodating reagents and samples on a transparent substrate. In addition, the PCR device is provided with a camera for capturing fluorescent light generated from the fluorescent dye combined with the target nucleic acids.

Meanwhile, detection of multiple target genes in one experiment is referred to as simultaneous multiplex detection, that is, multiplex PCR. For such multiplex PCR, various types of fluorescent dyes should be used, so a complex probe design in which a primer set composed of several primers and various fluorescent dyes are distinguished from each other and interference between the fluorescent dyes may be prevented is required. The reason is that wavelength bands for excitation and emission depending on various fluorescent colors overlap with each other.

Recently, in order to solve the problem of a chemical multiplex method, a spatially isolated system has been developed. That is, as disclosed in a patent document, in a conventional multiplex PCR chip, a plurality of probes is fixed in a single reaction chamber, and a reaction between a sample and a probe particle fixed in the reaction chamber is induced, so that a plurality of target nucleic acids is amplified and detected at once. That is, the probes with different types are respectively fixed to different positions on the reaction chamber and are spatially separated from each other, and the primers specifically hybridizing with sequences of different nucleic acid molecules are respectively injected into the spatially separated probes, so that fluorescent colors emitted from the probes are analyzed on the basis of the positions of the probes, thereby enabling simultaneous multiplex detection. In this way, a size of optical equipment may be reduced, equipment cost may be lowered, and efficiency such as reducing the detection time may be improved.

However, when the conventional multiplex PCR chip fixes the spatially separated probes on the reaction chamber, that is, when a few drops of probes in a liquid state containing primers are applied to be fixed on the reaction chamber, there is a problem that it is difficult to form appropriate probe particles due to spread of the probe droplets. In addition, when the probe particles are formed, there is a problem that the probe particles easily fall off. In addition, there is a problem that the number of detectable target genes is limited because there is a limit in obtaining various combinations by using only the positions of the probes, and also there is a problem that simultaneous multiplex detection is not easy because the positions of the probes change when left and right sides of the PCR chip are interchanged with each other.

### Documents of Related Art

Korean Patent No. 10-1724281 (Registration Date: April 3, 2017)

### SUMMARY OF THE INVENTION

The present invention has been disclosed to solve these problems, and a main objective of the present invention is to provide a multiplex PCR chip capable of simultaneously amplifying and detecting multiple target nucleic acids at once.

Another objective of the present invention is to provide a multiplex PCR chip capable of spatially isolating a plurality of probe particles specifically hybridizing with different nucleic acid molecules and to provide a multiplex PCR chip capable of enabling simultaneous multiplex detection on the basis of positions of the probe particles.

Yet another objective of the present invention is to provide a PCR chip capable of easily providing sizes and shapes of probe particles by forming spatially separated probe particles inside reaction chambers of the PCR chip, and forming particle-forming grooves accommodating a predetermined amount of a probe inside the reaction chambers.

In addition, still another objective of the present invention is to provide a PCR chip in which a particle holder composed of a plurality of protrusions protruding a predetermined length is formed on the bottom and inner surface of a particle-forming groove configured to form a probe particle, whereby the probe particle fixed to the particle-forming groove is fixed so as not to be easily separated.

In addition, still another objective of the present invention is to provide a multiplex PCR chip in which a plurality of probe particles specifically hybridizing with nucleic acid molecules is spatially isolated, and at the same time, sizes, shapes, or patterns of probe particles are made different from each other or shapes or patterns of particle holders are made different from each other, so that various types of target nucleic acids may be detected simultaneously on the basis of positions of the probe particles as well as the shapes and patterns of the probe holders.

In addition, still another objective of the present invention is to provide a multiplex PCR chip in which a plurality of reaction chambers is connected with a single connection channel when injecting a sample into the multiplex PCR chip so that all reaction chambers may be filled with the sample with one sample injection, and a waterproof coating is applied to surfaces of probe particles respectively fixed to the plurality of reaction chambers, so as to facilitate simultaneous multiplex detection.

In addition, still another objective of the present invention is to provide a multiplex PCR method using the above-described multiplex PCR chip.

As a means for achieving the objectives of the present invention, a multiplex PCR chip according to the present invention includes: a substrate made of a transparent plastic material; one or more reaction chambers formed on the substrate and configured to have a predetermined size and depth; and a plurality of particle-forming grooves formed in each reaction chamber and configured to have a size and depth capable of accommodating a predetermined amount of a probe in a liquid state, wherein the probe injected into spatially separated particle-forming grooves different from each other includes: primers having different types and specifically hybridizing with sequences of different nucleic acid molecules; a fluorescent dye combined with the primers; and a supporting body to which the primers are fixed.

As another exemplary embodiment of the present invention, a multiplex PCR chip according to the present invention includes: a substrate made of a transparent plastic material; one or more reaction chambers formed on the substrate and configured to have a predetermined size and depth; and a plurality of particle-forming grooves formed in each reaction chamber and configured to have a size and depth capable of accommodating a predetermined amount of a probe in a liquid state, wherein the particle-forming grooves have various planar shapes and the probes injected into the particle-forming grooves having different planar shapes includes: primers having different types and specifically hybridizing with sequences of different nucleic acid molecules; a fluorescent dye combined with the primers; and a supporting body to which the primers are fixed.

In the present invention, the supporting body included in the probe may form a three-dimensional reticular structure by a physical stimulus (i.e., light, temperature, and ultrasound waves) so that probe particles each having shapes respectively corresponding to the particle-forming grooves may be formed.

Particle holders may be respectively formed in the particle-forming grooves to prevent each probe particle from being separated.

Each particle holder may be composed of a plurality of protrusions protruding a predetermined length from a bottom or an inner surface of each particle-forming groove, and may be integrally combined with each probe particle.

The particle holders may be formed in different shapes or patterns, the particle holders respectively formed in the spatially separated particle-forming grooves may have shapes or patterns different from each other, and the probe injected into the particle-forming grooves may include different types of the primers that specifically hybridize with the sequences of different nucleic acid molecules.

A plurality of reaction chambers may be formed on the substrate, the plurality of reaction chambers may be connected to each other in series through a single connection channel, an inlet for injecting a sample may be formed at one end of the connection channel, and an outlet for discharging the sample may be formed at the other end thereof, whereby the sample injected through the inlet may be supplied to and filled in all reaction chambers.

In a multiplex PCR method according to the present invention, the multiplex PCR method includes: step S110 of preparing a PCR chip, wherein the PCR chip provided with a plurality of reaction chambers connected to each other in series through a single connection channel and provided with a plurality of spatially separated particle-forming grooves formed inside each reaction chamber; step S120 of injecting a probe, wherein the probe in a solution state is injected and provided with primers specifically hybridizing with sequences of different nucleic acid molecules in the plurality of particle-forming grooves, a fluorescent dye combined to the primers, and a supporting body forming a three-dimensional reticular structure when cured; step S130 of fixing probe particles, wherein a physical stimulus is applied to the probe injected into each particle-forming groove to cure the supporting body, and at the same time, the primers are fixed to an inner side of the supporting body to form the probe particles having shapes respectively corresponding to the particle-forming grooves; and step S140 of cleaning the probe particles, wherein pores are formed in the supporting body by injecting a cleaning solution into the plurality of reaction chambers to which the probe particles are respectively fixed.

The multiplex PCR method may further include: step S150 of seal, wherein a sealing film is attached to one surface of the PCR chip in order to seal open parts of the plurality of reaction chambers and the connection channel, and a sticker is attached to seal each opening of an inlet and outlet; step S160 of injecting a sample, wherein the sample is injected into and filled in the plurality of reaction chambers through the inlet; and step S170 of PCR, wherein, after specific nucleotide sequences included in the sample and the primers are hybridized by repeatedly heating and cooling the PCR chip and target nucleic acids are replicated and amplified in chains, excitation light may be emitted to pass through the plurality of reaction chambers, and a plurality of target genes may be detected at once on the basis of fluorescent colors emitted from the fluorescent dye combined with the primers and the positions and shapes of the probe particles.

Each particle holder composed of a plurality of protrusions protruding a predetermined length to fix each probe particle formed inside each particle-forming groove may be provided on an inner surface of each of the plurality of particle-forming grooves formed in the substrate.

The particle holders may be formed in different shapes or patterns, the particle holders respectively formed in the spatially separated particle-forming grooves may have shapes or patterns different from each other, and the probe injected into the particle-forming grooves may include different types of the primers that specifically hybridize with the sequences of different nucleic acid molecules.

According to the present invention, there is an effect that multiple target nucleic acids are simultaneously amplified and detected at once.

The present invention has an effect that a plurality of probe particles specifically hybridizing with different nucleic acid molecules may be spatially isolated, and simultaneous multiplex detection of the nucleic acid molecules may be performed on the basis of positions of the probe particles.

The present invention has an effect that particle-forming grooves accommodating a predetermined amount of the probe are formed inside a reaction chamber so that sizes and shapes of probe particles may be easily provided, and simultaneous multiplex detection may be performed on the basis of spatially separated positions and shapes of the probe particles.

The present invention has an effect that a particle holder composed of a plurality of protrusions protruding a predetermined length is formed on the bottom and inner surface of a particle-forming groove configured to form a probe particle, whereby the probe particle fixed to the particle-forming groove may be fixed so as not to be easily separated.

The present invention has an effect of providing a multiplex PCR chip in which a plurality of probe particles specifically hybridizing with nucleic acid molecules is spatially isolated, and at the same time, sizes, shapes, or patterns of probe particles are made different from each other or shapes or patterns of particle holders are made different from each other, so that various types of target nucleic acids may be detected simultaneously on the basis of positions of the probe particles as well as the shapes and patterns of the probe holders.

The present invention has an effect that a plurality of reaction chambers is connected with a single connection channel when injecting a sample into a PCR chip so that all reaction chambers may be filled with the sample with one sample injection, and a waterproof coating is applied to surfaces of probe particles respectively fixed to the plurality of reaction chambers so as to facilitate simultaneous multiplex detection.

### BRIEF DESCRIPTION OF THE DRAWINGS

FIG. 1 is a schematic plan view showing an example of a multiplex PCR chip according to the present invention.
FIG. 2 is a schematic cross-sectional view showing the multiplex PCR chip shown in FIG. 1.
FIGS. 3A to 3D are plan views showing various exemplary embodiments of the multiplex PCR chip according to the present invention.
FIG. 4 is a schematic cross-sectional view showing an example of a multiplex PCR device according to the present invention.
FIG. 5 is a schematic plan view showing another exemplary embodiment of a multiplex PCR chip according to the present invention.
FIG. 6 is a schematic plan view showing yet another exemplary embodiment of a multiplex PCR chip according to the present invention.
FIG. 7 is cross-sectional views illustrated with a flowchart showing a multiplex PCR method using the multiplex PCR chip shown in FIG. 6.

### DETAILED DESCRIPTION OF THE INVENTION

Advantages and features of the present invention, and a method for achieving the same, will be described with reference to the exemplary embodiments described below in detail in conjunction with the accompanying drawings. However, the present invention is not limited to the exemplary embodiments described herein and may be embodied in other forms. However, the present exemplary embodiment is provided to describe in detail enough to be able to easily implement the technical idea of the present invention to those skilled in the art to which the present invention belongs. Further, in the following description, if it is decided that the detailed description of a known function or configuration related to the invention makes the subject matter of the invention unclear, the detailed description is omitted.

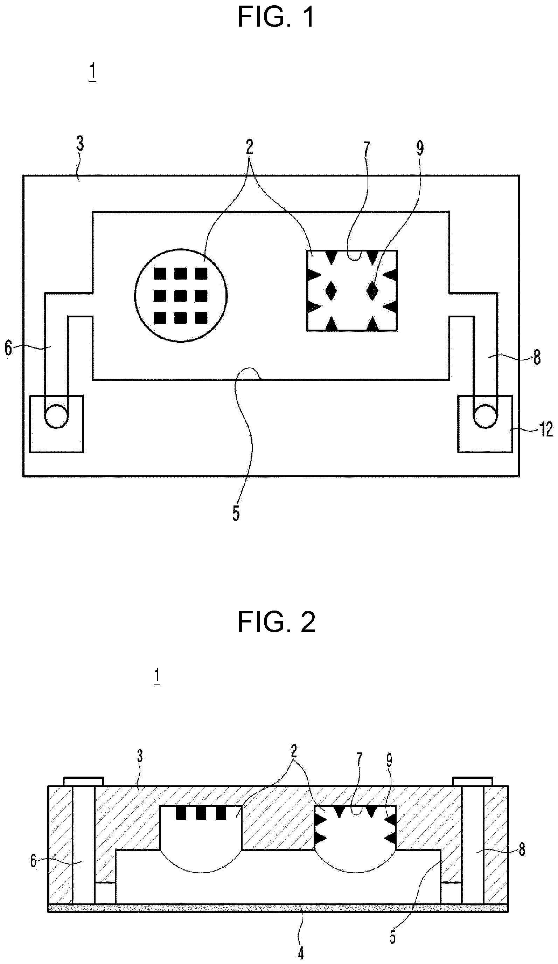

First, FIG. 1 is a plan view showing an example of a multiplex PCR chip according to the present invention, and FIG. 2 is a cross-sectional view showing the multiplex PCR chip shown in FIG. 1. As shown in FIGS. 1 and 2, in a multiplex PCR chip 1 of the present invention (hereinafter referred to as "PCR chip"), a plurality of probe particles 2 specifically hybridizing with nucleic acid molecules in a sample is spatially separated, and at the same time, shapes of the probe particles 2 are made different from each other so as to allow simultaneous multiplex detection to be performed with ease on the basis of the positions and shapes of the probe particles 2.

To this end, the multiplex PCR chip 1 of the present invention includes: a substrate 3 made of a transparent plastic material; one or more reaction chambers 5 formed on a surface of the substrate 3 and configured to have a predetermined size and depth; and one or more particle-forming grooves 7 formed on a bottom surface of each reaction chamber 5 and configured to have a predetermined size and depth.

In addition, a probe is injected into each particle-forming groove 7 and includes: primers specifically hybridizing with sequences of different nucleic acid molecules; a fluorescent dye combined with the primers; and a supporting body to which the primers are fixed. Accordingly, when the probe in a liquid state injected into the particle-forming groove 7 is cured, a probe particle 2 is formed in a shape corresponding to the particle-forming groove 7.

In this case, the particle-forming grooves 7 may be spatially separated and formed in different planar shapes. Then, the probe particles 2 respectively formed by the particle-forming grooves 7 are also spatially isolated and at the same time have different shapes.

In addition, a particle holder 9 composed of a plurality of protrusions protruding to a predetermined length so as to fix a probe particle 2 is formed on the bottom or inner surface of a particle-forming groove 7. The protrusions of the particle holder 9 may be formed in various shapes and the protrusions may be arranged in various patterns. Therefore, the probe particle 2 formed by the particle-forming groove 7 is integrated with the particle holder 9 to prevent separation, and at the same time, the particle holders 9 respectively formed in different particle-forming grooves 7 are configured in different shapes and patterns, so that target genes in various types may be distinguished from each other and detected thereby.

Meanwhile, an inlet 6 is formed on one side of the substrate 3 so that a sample may be injected into the reaction chamber 5, and an outlet 8 is formed on the opposite side of the substrate 3 so that the sample in the reaction chamber 5 may be discharged. In addition, a connection channel is formed between the inlet 6 and the outlet 8. In addition, a sealing film 4 is attached to a lower surface of the substrate 3. The sealing film 4 is made of a thin film to seal an open part of the reaction chamber 5 and simultaneously allow heat to be rapidly transferred to the sample inside the reaction chamber 5. In addition, the surface of the sealing film 4 is treated to be hydrophilic so that the sample may move smoothly. In addition, a sealing sticker 12 for sealing each opening of the inlet 6 and the outlet 8 may be further provided.

In this way, the multiplex PCR chip 1 of the present invention forms a plurality of spatially separated particle-forming grooves 7 inside the reaction chamber 5, spatially separates and forms the probe particles 2 respectively having a shape corresponding to the particle-forming grooves 7, and varies planar shapes of the particle-forming grooves 7, whereby the probe particles 2 having different shapes may be easily distinguished from each other.

In addition, in the present invention, a particle holder 9 composed of a plurality of protrusions protruding a predetermined length is formed at a bottom or an inner surface of the particle-forming groove 7 so as to prevent the probe particle 2 from being easily separated, and at the same time shapes and patterns of the protrusions constituting the particle holder 9 are variously formed, thereby enabling simultaneous multiplex detection of various types of target genes on the basis of not only the positions and/or shapes of the probe particles 2, but also the shapes and patterns of the particle holders 9.

More specifically, referring back to FIGS. 1 and 2, the substrate 3 has a predetermined thickness, and one or more reaction chambers 5 each having a predetermined size and depth are formed on a part of a surface of the substrate 3. In addition, at the bottom of each reaction chamber 5, a plurality of particle-forming grooves 7 is formed to be spaced apart from each other by a predetermined distance. Accordingly, the particle-forming grooves 7 are spatially separated from each other.

In this case, each particle-forming groove 7 has a size and depth that may accommodate a predetermined amount of a probe. The probe includes: primers specifically hybridizing with sequences of different nucleic acid molecules in a sample; a fluorescent dye combining with the primers and emitting fluorescent light of a predetermined wavelength; and a supporting body forming a three-dimensional reticular structure when cured while maintaining a liquid state and causing the primers to be fixed thereto.

The particle-forming grooves 7 are formed in various shapes. For example, the particle-forming grooves 7 are configured to have various planar shapes such as a square, a circle, a pentagon. Accordingly, when a predetermined amount of the probe is injected into each of the plurality of particle-forming grooves 7 and cured, the probe particles 2 are respectively formed in shapes corresponding to the particle-forming grooves 7.

In addition, on the inner surface of the particle-forming groove 7, the particle holder 9 composed of the plurality of protrusions protruding a predetermined length is integrally formed in order to fix the probe particle 2. For example, the particle holder 9 may be molded together when the substrate 3 is injection-molded. Preferably, when forming a probe particle 2, the particle holder 9 is integrally combined with the probe particle 2 by roughening a surface of the particle-forming groove 7 or increasing the surface area so that the probe particle 2 is prevented from being separated.

In addition, the protrusions constituting the particle holder 9 may be formed in various shapes such as a square, a circle, a pyramid, and the like. In addition, the protrusions formed on the bottom and/or the inner surface of the particle-forming groove 7 may be formed in various patterns.

As such, in the present invention, the particle-forming grooves 7 are formed so that the probe particles 2 may be easily formed, and at the same time, the planar shapes of the particle-forming grooves 7 are allowed to be varied, so that various types of probe particles 2 are formed, whereby the probe particles 2 may be spatially separated from each other and at the same time morphologically distinguished from each other. In addition, different types of primers specifically hybridizing with different nucleic acid molecules are injected into different particle-forming grooves 7, whereby a plurality of target genes may be detected by simultaneous multiplex detection on the basis of the shapes as well as the positions of the probe particles 2.

In addition, in the present invention, the plurality of spatially separated particle-forming grooves 7 is formed in a reaction chamber 5, and at the same time, by forming a shape of a particle holder 9 configured to have the plurality of protrusions protruding to fix the probe particle 2 on the inner surface of a particle-forming groove 7, the probe particle 2 is firmly fixed and also different types of primers specifically hybridizing with different nucleic acid molecules are injected according to the shapes and patterns of the particle holders 9, thereby enabling simultaneous multiplex detection of various types of target genes on the basis of not only the positions and shapes of the probe particles 2, but also the shapes and patterns of the particle holders 9 respectively formed in the probe particles 2.

Referring back to FIGS. 1 and 2, on the substrate 3 of the PCR chip 1, the reaction chamber 5 is formed with a predetermined depth and size so as to accommodate a predetermined amount of the sample. In addition, two particle-forming grooves 7 are formed at the bottom of the reaction chamber 5. In this case, the two particle-forming grooves 7 are installed to be spaced apart from each other by a predetermined distance and are spatially separated from each other. For example, to form the probe particles 2, a probe containing different kinds of primers that specifically hybridize with different nucleic acid molecules is injected into the particle-forming grooves 7 on the right and left sides in FIGS. 1 and 2.

In addition, the particle-forming grooves 7 are formed with planar shapes different from each other. For example, the particle-forming groove 7 on the left side has a circular planar shape, and the particle-forming groove 7 on the right side has a rectangular planar shape. In addition, probe particles 2 are formed by injecting the probe containing different types of the primers into the particle-forming grooves 7 having different planar shapes.

Meanwhile, a supporting body included in a probe particle 2 serves to support the probe particle 2 while at the same time fixing the primers. Preferably, the supporting body is made of a material that is maintained in a liquid state so as to be mixed with the primers and may be converted into a gel or solid state so as to cause the probe particle 2 to be formed after being injected into the particle-forming groove 7.

Preferably, the supporting body may be made of a porous sponge or hydrogel that forms a three-dimensional reticular formation. Hydrogel refers to a material having a three-dimensional hydrophilic polymer reticular structure that may contain a large amount of moisture. The components of the hydrogel include polyvinyl alcohol, sodium polyacrylate, acrylate polymers, and copolymers having abundant hydrophilic groups. Since greater than or equal to 90% of the components of the hydrogel are water and have fluidity, primers and the like may be mixed. Such hydrogels may exhibit various properties depending on which polymer is selected as a main chain or what cross-linking method is selected, and adhesiveness may be introduced into the hydrogel by using the properties of the polymer chain itself or by introducing a specific functional group into the polymer chain.

In addition, when hydrogel receives a physical stimulus such as light, heat, or ultrasound waves, the hydrogel is easily cured and changed into a gel form. That is, when the hydrogel receives the physical stimulus, the hydrogel forms a three-dimensional reticular formation. For example, when PEGDA pre-polymer solution is illuminated with ultraviolet light several times with an interval of 2 seconds, a hydrogel fixture may be fixed on a plastic substrate as well. Therefore, while applying the physical stimulus to the hydrogel, primers are dispersed and fixed inside the supporting body.

Therefore, when a predetermined physical stimulus is given after injecting a probe into the particle-forming groove 7, the supporting body is cured while forming a reticulated porous body to form the probe particle 2. Then, when the probe particle 2 is cleaned with washing water, impurities in the passage between the porous bodies are washed away to form a porous probe particle. Accordingly, in the probe particle 2, numerous passages through which the sample may pass are formed. In addition, the sample inserted into the passages of the probe particle 2 reacts with a specific primer in the probe particle 2. In addition, a fluorescent dye included in the probe particle 2 emits fluorescent light of a specific wavelength in response to excitation light emitted from the outside.

In addition, since a probe particle 2 is formed in a shape corresponding to a particle-forming groove 7, the probe particle 2 may be easily separated from the particle-forming groove 7 in general. In order to solve this problem, a particle holder 9 composed of a plurality of protrusions protruding a predetermined length on an inner surface, that is, the bottom and the inner surface of the particle-forming groove 7 is formed. Then, the plurality of protrusions are integrally coupled to an inner side of the probe particle 2 so as to prevent the probe particle 2 from being separated.

In addition, the protrusions of the particle holder 9 formed in the particle-forming groove 7 may be formed in various shapes such as a cylinder, a square pillar, and a diamond. In addition, the plurality of protrusions formed on the bottom and inner surface of the particle-forming groove 7 may be arranged in various patterns.

In this way, the particle holder 9 not only improves the combining capability with probe particle 2, but also allows the probe particle 2 to be visually distinguished. For example, when the probe particle 2 is photographed, the shape and pattern of the particle holder 9 coupled to the inside of the probe particle 2 may be visually distinguished. Accordingly, simultaneous multiplex detection of various types of nucleic acid molecules is possible on the basis of the shape and pattern of the particle holder 9 formed on the probe particle 2.

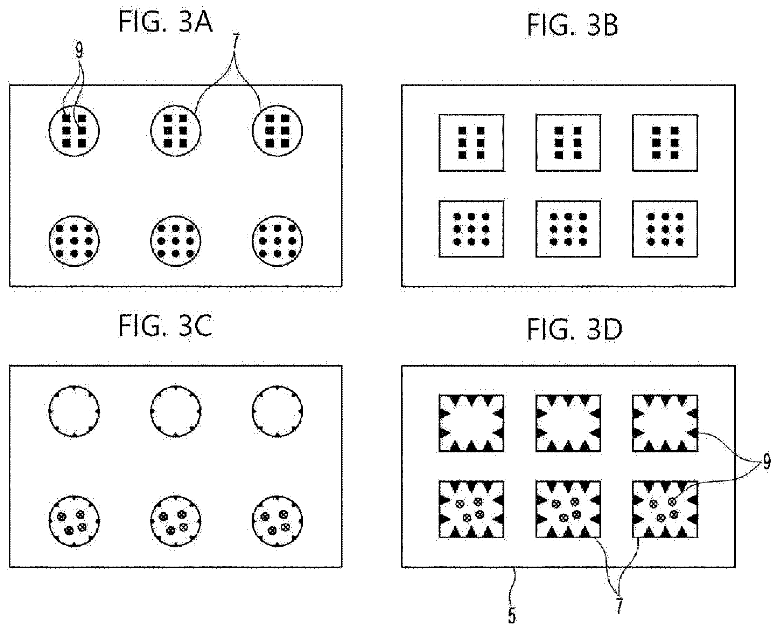

Next, FIGS. 3A to 3D are plan views showing various types of PCR chips according to the present invention.

As shown, in the PCR chip 1 of FIG. 3A, six circular particle-forming grooves 7 in one reaction chamber 5 are arranged in two rows to be spaced apart at a predetermined interval. In addition, a particle holder 9 composed of rectangular protrusions is formed at the bottom of each particle-forming groove 7 arranged at the upper side of the reaction chamber 5, and a particle holder 9 composed of circular protrusions is formed at the inside of each particle-forming groove 7 arranged at the lower side of the reaction chamber 5. Therefore, by forming the probe particles 2 including different primers according to the positions of the particle-forming grooves 7 and the shapes of the particle holders 9, simultaneous multiplex detection is possible.

Next, in the PCR chip 1 of FIG. 3B, six rectangular particle-forming grooves 7 in one reaction chamber 5 are arranged in two rows up and down to be spaced apart at a predetermined interval, wherein the particle holder 9 composed of rectangular protrusions is formed at the bottom of each particle-forming groove 7 arranged at the upper side of the reaction chamber 5, and the particle holder 9 composed of circular protrusions is formed at the inside of each particle-forming groove 7 arranged at the lower side of the reaction chamber 5. Therefore, by forming the probe particles 2 including different primers according to the positions of the particle-forming grooves 7 and the shapes of the particle holders 9, simultaneous multiplex detection is possible.

Next, in the PCR chip 1 of FIG. 3C, six circular particle-forming grooves 7 in one reaction chamber 5 are arranged in two rows up and down to be spaced apart at a predetermined interval, wherein the particle holder 9 composed of circular protrusions protruding horizontally for a predetermined length is formed at the inner surface of each particle-forming groove 7 arranged at the upper side of the reaction chamber 5, and the particle holder 9 composed of circular protrusions protruding vertically for a predetermined length is formed at the bottom of each particle-forming groove 7 arranged at the lower side of the reaction chamber 5. Therefore, by forming the probe particles 2 including different primers according to the positions of the particle-forming grooves 7 and the shapes and patterns of the particle holders 9, simultaneous multiplex detection is possible.

In addition, in the PCR chip 1 of FIG. 3D, six rectangular particle-forming grooves 7 in one reaction chamber 5 are arranged in two rows up and down to be spaced apart at a predetermined interval, wherein the particle holder 9 composed of triangular protrusions protruding horizontally for a predetermined length is formed at the inner surface of each particle-forming groove 7 arranged at the upper side of the reaction chamber 5, and the particle holder 9 composed of angular protrusions protruding vertically for a predetermined length is formed at the bottom of each particle-forming groove 7 arranged at the lower side of the reaction chamber 5. Therefore, by forming the probe particles 2 including different primers according to the positions of the particle-forming grooves 7 and the shapes and patterns of the particle holders 9, simultaneous multiplex detection is possible.

As such, the present invention forms the plurality of probe particles 2 to be spatially separated, wherein the shapes of the probe particles 2 are made different from each other, and the shapes and patterns of the particle holders 9 formed inside of the probe particles 2 are made different from each other, thereby distinguishing various types of nucleic acid sequences and enabling simultaneous multiplex detection.

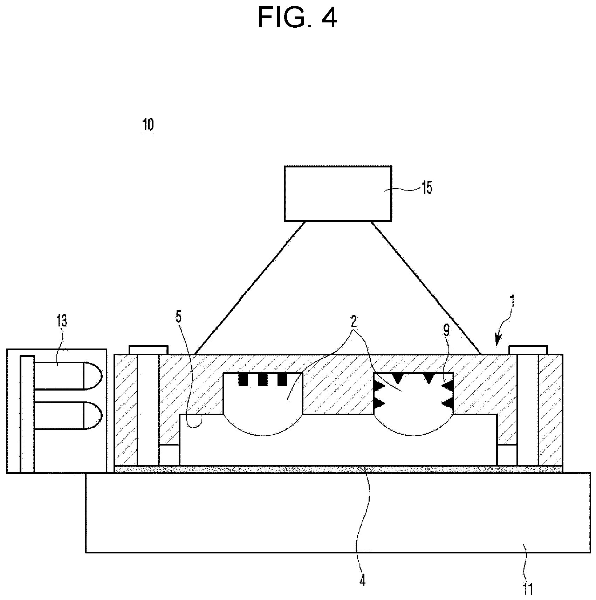

Next, FIG. 4 is a schematic cross-sectional view showing a preferred structure of a multiplex PCR device 10 according to the present invention. As shown, the multiplex PCR device 10 of the present invention mainly includes a heating block 11, a light source part 13, and a camera 15. The heating block 11 heats and cools the PCR chip 1 at predetermined time intervals. To this end, the PCR chip 1 is seated in close contact with an upper surface of the heating block 11. In addition, the light source part 13 is installed on one or both sides of the PCR chip 1. The light source part 13 emits excitation light having a predetermined wavelength to pass through the reaction chambers 5 formed in the PCR chip 1. Preferably, the light source part 13 may be formed with a plurality of LEDs. In addition, the camera 15 is arranged to capture the fluorescent light emitted from the plurality of probe particles 2. That is, when excitation light of a specific wavelength is emitted to the probe particle 2, the fluorescent dye combined with the primers reacts to emit fluorescence of a specific wavelength. Accordingly, when the fluorescent light emitted from the probe particle 2 is captured by using the camera 15, the target gene may be distinguished by using fluorescent colors.

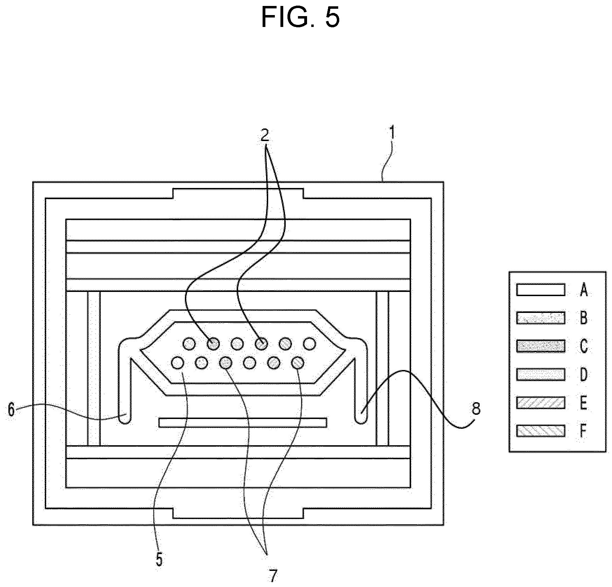

Meanwhile, FIG. 5 is a plan view showing another exemplary embodiment of a multiplex PCR chip according to the present invention. As shown, the multiplex PCR chip 1 includes: a substrate 3 made of a light-transmitting plastic; a reaction chamber 5 formed on the substrate 3; a probe particle 2 formed at a predetermined interval inside the reaction chamber 5; and an inlet 6 and an outlet 8 connected to the reaction chamber 5. In this case, the probe particles 2 may be formed by the plurality of particle-forming grooves 7 formed to be spaced apart from each other at a predetermined interval inside the reaction chamber 5. In this way, since the plurality of probe particles 2 is spatially separated and includes different primers, the fluorescent colors emitted from each probe particle 2 may be detected, whereby simultaneous multiplex detection of multiple target genes at once is possible.

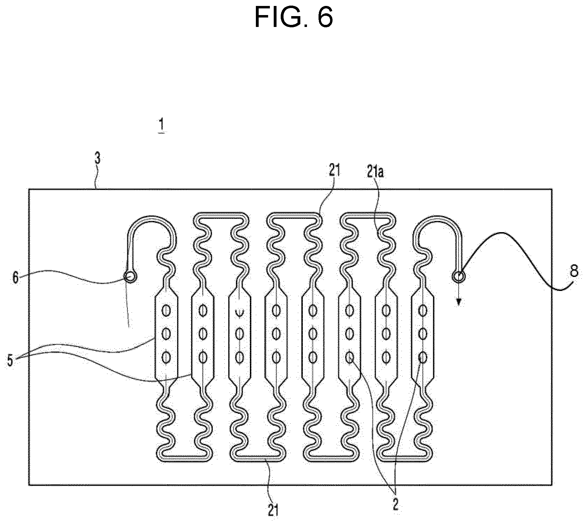

Next, FIG. 6 is a plan view showing another exemplary embodiment of a multiplex PCR chip according to the present invention. As shown, the multiplex PCR chip 1 includes: a substrate 3 made of a light-transmitting plastic; a plurality of reaction chambers 5 formed on the substrate 3; a plurality of probe particles 2 respectively formed in the reaction chambers 5 at a predetermined interval; a connection channel 21 configured to connect the plurality of reaction chambers 5 to each other in series; and an inlet 6 and an outlet 8 respectively formed at opposite ends of the connection channel 21. In this case, the probe particles 2 may be formed by the plurality of particle-forming grooves 7 formed to be spaced apart from each other at a predetermined interval inside the reaction chamber 5. In addition, the particle holder 9 composed of the plurality of protrusions may be integrally formed on an inner surface of the particle-forming groove 7.

As such, the multiplex PCR chip of the exemplary embodiment connects the plurality of reaction chambers 5 to each other in series by using a single connection channel 21, so that the sample injected through the inlet 6 is supplied to all the reaction chambers 5 and filled therein, thereby eliminating repetitive pipetting and facilitating simultaneous multiplex detection. Preferably, a plurality of bent parts 21a may be formed in the connection channel 21 to suppress movement of the sample between adjacent reaction chambers 5 to some extent.

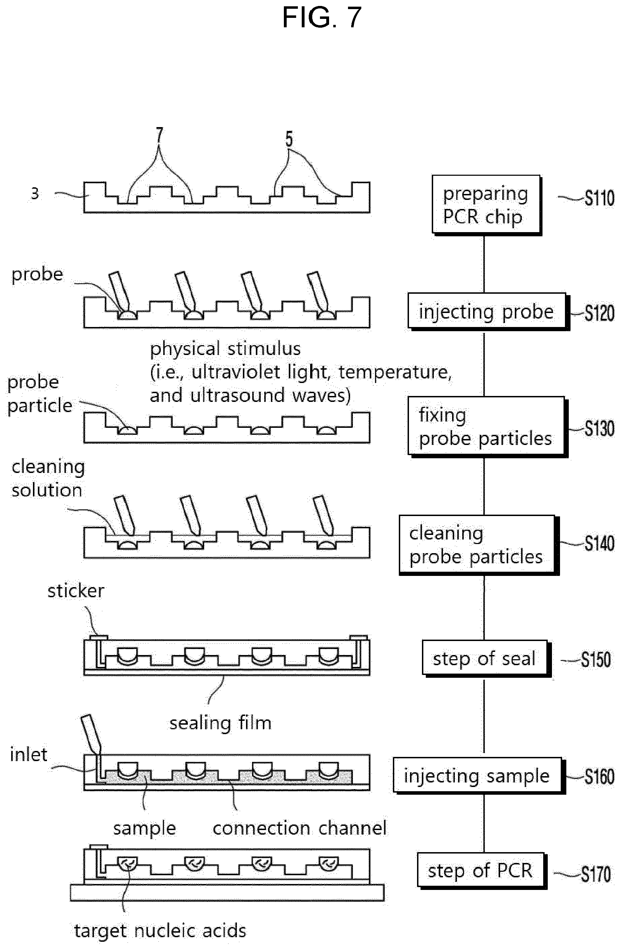

FIG. 7 is cross-sectional views illustrated with a flowchart showing a multiplex PCR method using the multiplex PCR chip shown in FIG. 6.

As shown, the multiplex PCR method according to the present invention includes: step S110 of preparing a PCR chip; step S120 of injecting a probe; step S130 of fixing probe particles; and step S140 of cleaning the probe particles. In addition, the multiplex PCR method further includes: step S150 of seal, step S160 of injecting a sample; and step S170 of PCR. In addition, after step S140 of cleaning the probe particles, step of drying the probe particles may be further included, wherein the cleaned probe particles are dried. In this way, when the probe particles are dried, it is easy to store and transport the PCR chip, and also contamination of the probe particles may be prevented during storage or transport.

Specifically, step S110 of preparing a PCR chip is a step in which a plurality of reaction chambers 5 is formed, a plurality of particle-forming grooves 7 is formed at a predetermined interval inside each reaction chamber 5, a plurality of reaction chambers 5 is connected to each other in series through a single connection channel 21, an inlet 6 is formed at one end of the connection channel 21 and an outlet 8 is formed at the other end thereof. In this case, the particle-forming grooves 7 are spatially separated from each other and respectively formed in different shapes so as to be visually distinguished, and the particle holder 9 may be integrally formed inside each particle-forming groove 7. In addition, the particle holders 9 may be formed in different shapes or patterns.

Next, step S120 of injecting a probe is a step in which a probe including primers, a fluorescent dye, and a supporting body is injected into the plurality of particle-forming grooves 7 formed inside the reaction chamber 5 of the PCR chip 1. In this case, the primers specifically hybridizing with different nucleic acid molecules may be injected into the spatially separated particle-forming grooves 7. In addition, the fluorescent dye emits fluorescent light of a predetermined wavelength in response to excitation light emitted from the outside. In addition, the supporting body may be made of a hydrogel having a three-dimensional hydrophilic polymer reticular structure. The hydrogel is made in a liquid state and may be mixed with the primers and is cured when subjected to a physical stimulus, thereby forming a porous structure.

In step S130 of fixing probe particles, a predetermined physical stimulus is applied to the probe injected into the particle-forming grooves 7 so that the supporting body is transformed into a three-dimensional reticular structure, and at the same time, the primers are allowed to be fixed inside the reticular structure. For example, when the probe is heated at a predetermined temperature or illuminated with ultraviolet light, the hydrogel forms a three-dimensional reticular formation, and the primers are fixed to the inside of the reticular formation. In this case, the probe is fixed in a shape corresponding to the particle-forming groove 7 to form a probe particle 2. In addition, in the process of forming the probe particle 2, the protrusions of the particle holder 9 are integrally coupled to the inside of the probe particle 2.

Subsequently, in step S140 of cleaning the probe particles, washing water is injected into the plurality of reaction chambers 5 to remove impurities trapped in the pores of the probe particles 2, so as to ensure porosity. Accordingly, the porous probe particle 2 may react with the primers by introducing the sample along the plurality of passages.

Next, in step S150 of seal, a sealing film is attached to one surface of the PCR chip 1 to seal open parts of the plurality of reaction chambers 5 and the connection channel 21, and a sticker is attached to an opening of the inlet 6 or outlet 8, thereby sealing the whole of the PCR chip 1. The above steps may be performed by an expert indoors, such as an expert in a laboratory, and subsequent steps may be performed at a site where a blood sample may be collected.

Step S160 of injecting a sample is a step in which the sample is injected into the plurality of reaction chambers 5. In this case, in a case where the plurality of reaction chambers 5 is connected to each other in series through the connection channel 21 and an inlet 6 is formed at one end of the connection channel 21 and an outlet 8 is formed at the other end thereof, when a sample is injected into the inlet 6, the sample is injected into the plurality of reaction chambers 5 through the connection channel 21, so that all of the reaction chambers 5 may be filled with the sample through one pipetting. In this case, since the probe particles 2 formed in the reaction chamber 5 are coated with a waterproof coating agent, the probe particles 2 are prevented from melting or from moving to be mixed with different primers.

Finally, in step S170 of PCR, after the PCR chip 1 is repeatedly heated and cooled to replicate and amplify target nucleic acid molecules in chains, excitation light is emitted to penetrate the reaction chambers 5 of the PCR chip 1 in order to detect the amplified product, the fluorescent light having a specific wavelength emitted by the fluorescent dye included in the plurality of probe particles 2 in response to the emitted excitation light is captured by the camera 15, and the fluorescent colors are analyzed, thereby performing simultaneous multiplex detection of various target genes at once.

As such, the multiplex PCR chip 1 of the exemplary embodiment has an effect that the plurality of probe particles specifically hybridizing with different nucleic acid molecules may be spatially isolated, and simultaneous multiplex detection of the nucleic acid molecules may be performed on the basis of the positions of the probe particles. In addition, the present invention has an effect that the particle-forming grooves accommodating a predetermined amount of the probe are formed inside the reaction chamber so that the sizes and shapes of the probe particles may be easily provided, and simultaneous multiplex detection may be performed on the basis of spatially separated positions and shapes of the probe particles.

In addition, the present invention has an effect that the particle holder composed of the plurality of protrusions protruding a predetermined length is formed on the bottom and inner surface of each particle-forming groove configured to form the probe particle so that the probe particle fixed to the particle-forming groove is fixed so as not to be easily separated. In addition, the present invention has an effect of providing the multiplex PCR chip in which the plurality of probe particles specifically hybridizing with nucleic acid molecules is spatially isolated while at the same time, sizes, shapes, or patterns of probe particles are made different from each other, or shapes or patterns of particle holders are made different from each other, so that simultaneous multiplex detection of multiple types of target nucleic acids may be performed on the basis of not only the positions of the probe particles, but also the shapes and patterns of the probe holders.

In the above specification and drawings, preferred exemplary embodiments of the present invention has been disclosed, and although specific terms have been used, these terms are only used in a general sense to easily explain the technical contents of the present invention and to help the understanding of the present invention, and is not intended to limit the scope of the present invention. In addition, the present invention is not limited to the exemplary embodiments disclosed above, but may be implemented in various different forms. These exemplary embodiments are provided only to complete the disclosure of the present invention and to completely inform the scope of the present invention to those skilled in the art to which the present invention pertains, and the present invention is only defined by the scope of the claims.

## Claims

1. A multiplex PCR chip, comprising:
a substrate made of a transparent plastic material;
one or more reaction chambers formed on the substrate and configured to have a predetermined size and depth; and
a plurality of particle-forming grooves formed in each reaction chamber and configured to have a size and depth capable of accommodating a predetermined amount of a probe in a liquid state,
wherein the probe injected into spatially separated particle-forming grooves different from each other comprises:
primers having different types and specifically hybridizing with sequences of different nucleic acid molecules;
a fluorescent dye combined with the primers; and
a supporting body to which the primers are fixed.

2. A multiplex PCR chip, comprising:
a substrate made of a transparent plastic material;
one or more reaction chambers formed on the substrate and configured to have a predetermined size and depth; and
a plurality of particle-forming grooves formed in each reaction chamber and configured to have a size and depth capable of accommodating a predetermined amount of a probe in a liquid state,
wherein the particle-forming grooves have various planar shapes and
the probes injected into the particle-forming grooves having different planar shapes comprises:
primers having different types and specifically hybridizing with sequences of different nucleic acid molecules;
a fluorescent dye combined with the primers; and
a supporting body to which the primers are fixed.

3. The multiplex PCR chip of claim 1 or 2, wherein the supporting body included in the probe forms a three-dimensional reticular structure by a physical stimulus (i.e., light, temperature, and ultrasound waves) so that probe particles each having shapes respectively corresponding to the particle-forming grooves are formed.

4. The multiplex PCR chip of claim 3, wherein particle holders are respectively formed in the particle-forming grooves to prevent each probe particle from being separated.

5. The multiplex PCR chip of claim 4, wherein each particle holder is composed of a plurality of protrusions protruding a predetermined length from a bottom or an inner surface of each particle-forming groove, and is integrally combined with each probe particle.

6. The multiplex PCR chip of claim 4, wherein the particle holders are formed in different shapes or patterns,
the particle holders respectively formed in the spatially separated particle-forming grooves have shapes or patterns different from each other, and
the probe injected into the particle-forming grooves comprises different types of the primers that specifically hybridize with the sequences of different nucleic acid molecules.

7. The multiplex PCR chip of claim 1 or 2, wherein a plurality of reaction chambers is formed on the substrate,
the plurality of reaction chambers is connected to each other in series through a single connection channel,
an inlet for injecting a sample is formed at one end of the connection channel, and
an outlet for discharging the sample is formed at the other end thereof, whereby the sample injected through the inlet is supplied to and filled in all reaction chambers.

8. A multiplex PCR method, comprising:
step (S110) of preparing a PCR chip, wherein the PCR chip provided with a plurality of reaction chambers connected to each other in series through a single connection channel and provided with a plurality of spatially separated particle-forming grooves formed inside each reaction chamber;
step (S120) of injecting a probe, wherein the probe in a solution state is injected and provided with primers specifically hybridizing with sequences of different nucleic acid molecules in the plurality of particle-forming grooves, a fluorescent dye combined to the primers, and a supporting body forming a three-dimensional reticular structure when cured;
step (S130) of fixing probe particles, wherein a physical stimulus is applied to the probe injected into each particle-forming groove to cure the supporting body, and at the same time, the primers are fixed to an inner side of the supporting body to form the probe particles having shapes respectively corresponding to the particle-forming grooves; and
step (S140) of cleaning the probe particles, wherein pores are formed in the supporting body by injecting a cleaning solution into the plurality of reaction chambers to which the probe particles are respectively fixed.

9. The multiplex PCR method of claim 8, further comprising:
step (S150) of seal, wherein a sealing film is attached to one surface of the PCR chip in order to seal open parts of the plurality of reaction chambers and the connection channel, and a sticker is attached to seal each opening of an inlet and outlet;
step (S160) of injecting a sample, wherein the sample is injected into and filled in the plurality of reaction chambers through the inlet; and
step (S170) of PCR, wherein, after specific nucleotide sequences included in the sample and the primers are hybridized by repeatedly heating and cooling the PCR chip and target nucleic acids are replicated and amplified in chains, excitation light is emitted to pass through the plurality of reaction chambers, and a plurality of target genes is detected at once on the basis of fluorescent colors emitted from the fluorescent dye combined with the primers and the positions and shapes of the probe particles.

10. The multiplex PCR method of claim 8, further comprising:
a step of drying the probe particles, wherein the probe particles cleaned in step (S140) of cleaning the probe particles are dried.

11. The multiplex PCR method of claim 8, wherein each particle holder composed of a plurality of protrusions protruding a predetermined length to fix each probe particle formed inside each particle-forming groove is provided on an inner surface of each of the plurality of particle-forming grooves formed in the substrate.

12. The multiplex PCR method of claim 11, wherein the particle holders are formed in different shapes or patterns,
the particle holders respectively formed in the spatially separated particle-forming grooves have shapes or patterns different from each other, and
the probe injected into the particle-forming grooves comprises different types of the primers that specifically hybridize with the sequences of different nucleic acid molecules.
